# EUROPÄISCHE PATENTSCHRIFT

(11) **EP 2 379 488 B1**
(45) Veröffentlichungstag und Bekanntmachung des Hinweises auf die Patenterteilung: **20.02.2013**
(21) Anmeldenummer: 09799075.8
(22) Anmeldetag: 10.12.2009
(51) Int. Cl.: C07C 213/10, C07C 215/12

(54) **VERFAHREN ZUR HERSTELLUNG VON REINEM TRIETHANOLAMIN (TEOA)**
METHOD FOR PRODUCING PURE TRIETHANOLAMINE (TEOA)
PROCÉDÉ DE FABRICATION DE TRIÉTHANOLAMINE PURE (TEOA)

(30) Priorität: 19.12.2008 EP 08172435
(43) Veröffentlichungstag der Anmeldung: 26.10.2011
(73) Patentinhaber: BASF SE, 67056 Ludwigshafen (DE)
(72) Erfinder: MELDER, Johann-Peter, 67459 Böhl-Iggelheim (DE); CAUWENBERGE, Gunther van, B-9160 Lokeren (BE); FREMAU, Tom, B-2930 Brasschaat (BE); MOORS, Jürgen, B-3010 Kessel-Lo (BE); HAHN, Thilo, 67292 Kirchheimbolanden (DE); STEIN, Bernd, 64665 Alsbach-Hähnlein (DE)
(86) Internationale Anmeldenummer: PCT/EP2009/066839
(87) Internationale Veröffentlichungsnummer: WO 2010/069856

(56) Entgegenhaltungen:
- EP-A1- 1 443 036
- WO-A2-2005/035481
- US-A- 3 849 262

## Beschreibung

Die Erfindung betrifft ein Verfahren zur Herstellung von reinem TEOA durch kontinuierliche destillative Auftrennung eines Ethanolamingemisches enthaltend Diethanolamin (DEOA) und TEOA, wobei in einer Destillationskolonne (DEOA-Kolonne) DEOA abdestilliert wird und der anfallende Sumpfstrom enthaltend TEOA einer nachgeschaltenen Kolonne (TEOA-Kolonne) zugeführt wird, in der das reine TEOA als Seitenabzugsstrom entnommen wird.

Es ist allgemein bekannt, dass ein nach einer fraktionierenden Destillation eines TEOA-Rohprodukts, das durch Umsetzung von wässrigem Ammoniak mit Ethylenoxid und Abdestillieren von Monoethanolamin (MEOA) und Diethanolamin (DEOA) gewonnen wurde, erhaltenes und zunächst farbloses, reines TEOA (Farbzahl: ca. 0 bis 20 APHA nach DIN-ISO 6271 (= Hazen)), sich nach einer Lagerzeit von ca. 4 bis 6 Wochen, auch im geschlossenen Gebinde und unter Lichtausschluss, allmählich leicht rosa und schließlich, besonders leicht beim Stehen am Licht, gelb bis braun verfärben kann. Dieser Effekt wird durch Einwirkung von höheren Temperaturen beschleunigt. (Siehe z. B.: G.G. Smirnova et al., J. of Applied Chemistry of the USSR 61, Seiten 1508-9 (1988), und Chemical & Engineering News 1996, Sept. 16, Seite 42, mittlere Spalte).

Gemäß Chemical & Engineering News 1996, Sept. 16, Seite 42, zersetzt sich bei erhöhter Temperatur ein Mol TEOA in ein Mol Ethanolamin und zwei Mol Acetaldehyd. Acetaldehyd kondensiert zu Crotonaldehyd, der wiederum mit Ethanolamin eine Schiff'sche Base bildet. Diese ungesättigte Schiff'sche Base führt unter 1,4-Polymerisation zu farbigen Produkten im TEOA.

Zur Beurteilung der Farbqualität vom reinem TEOA hat sich neben den zeitaufwendigen Lagerversuchen, bei denen die APHA-Farbzahl (nach DIN-ISO 6271) des TEOAs in Abhängigkeit von der Lagerzeit gemessen wird, der sogenannte Säureneutralisationstest bewährt.

Dieser Säureneutralisationstest erlaubt die Beurteilung der farblichen Lagerstabilität von frisch hergestelltem TEOA innerhalb weniger Minuten.

Der Test wird in den japanischen Dokumenten JP 62 019 558 A (Derwent Abstract Nr. 87-067647/10) und JP 62 005 939 A (Derwent Abstract Nr. 87-047397/07) beschrieben, wonach das TEOA mit Essigsäure, Zitronensäure, Schwefelsäure, Salzsäure oder Phosphorsäure behandelt (neutralisiert) wird und danach die Extinktion der Absorptionsbanden bei 420 nm und 530 nm gemessen wird. Tritt während der Durchführung des Tests keine augenscheinliche Rosaverfärbung des TEOAs auf und bleiben die gemessenen Werte für die Extinktion genügend klein, so ist das TEOA farblich lagerstabil, bleibt also über einen Zeitraum von mehreren Monaten farblos.

In der Literatur sind verschiedene Verfahren zur Herstellung von reinem und farblosem bis wenig gefärbtem TEOA beschrieben.

EP 4015 A (BASF AG) beschreibt, dass Ethanolamine mit geringerer Verfärbung durch Zusatz von phosphoriger oder unterphosphoriger Säure während der Herstellung der Ethanolamine und/oder der destillativen Aufarbeitung erhalten werden.

EP 36 152 A und EP 4015 A (beide BASF AG) erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US 3,819,710 offenbart ein Verfahren zur Verbesserung der Farbqualität von Ethanolaminen durch Hydrierung der rohen Ethanolamine in Gegenwart ausgewählter Katalysatoren. Das Verfahren ist jedoch technisch aufwendig und führt nicht zu einer TEOA-Ware, die über mehrere Monate farblos bleibt.

US 3,207,790 beschreibt ein Verfahren zur Verbesserung der Farbqualität von Alkanolaminen durch Zugabe eines Borhydrids eines Alkalimetalls.

US 3,742,059 und DE 22 25 015 A beschreiben die Verbesserung der Farbqualität von Alkanolaminen durch den Zusatz eines Alkanolaminesters der Borsäure bzw. Alkali-/Erdalkalimetallboraten.

Die Anwesenheit eines Hilfsstoffes zur Stabilisierung von TEOA ist jedoch in vielen wichtigen Anwendungsbereichen des TEOAs unerwünscht.

Die nachträgliche Zugabe kleiner Mengen Ethylenoxid zu frisch destilliertem TEOA führt nach US 4,673,762 ebenfalls zu einer Entfärbung und Farbstabilisierung. Die Methode erscheint jedoch aus toxikologischen Gründen bedenklich.

GB 1 062 730 A beschreibt ein Verfahren zur Reinigung von Ethanolaminen durch Reindestillation in Gegenwart von Silikaten oder Aluminaten.

JP 62 019 558 A (Derwent Abstract Nr. 87-067647/10) berichtet über die Herstellung von qualitativ gutem TEOA durch Behandlung von rohem TEOA mit anorganischen Oxiden bei 170 bis 250 °C und anschließender Destillation in Abwesenheit von Sauerstoff.

Ähnliche Ergebnisse werden gemäß JP 62 005 939 A (Derwent Abstract Nr. 87-047397/07) erzielt, wenn rohes TEOA unter Luftausschluss 1 bis 10 h auf 170 bis 250 °C erhitzt und dann im Vakuum destilliert wird.

SU 326 178 A (Derwent Abstract Nr. 63384T-AE) beschreibt die Herstellung TEOA mit guter Farbqualität durch schonende Umsetzung von wasserfreiem Monoethanolamin (MEOA) oder Diethanolamin (DEOA) oder Mischungen beider Substanzen mit Ethylenoxid bei Temperaturen kleiner 50 °C.

Ähnliche Ergebnisse werden gemäß SU 228 693 A (Chem. Abstr. 70, 77305f (1969)) und GB 1 092 449 A erzielt, wenn Ammoniak mit Ethylenoxid bei kleiner/gleich 35 °C umgesetzt und die erhaltene Ethanolamin-Mischung unter Luftausschluss destilliert wird.

Vom wirtschaftlichen Gesichtspunkt sind solche Verfahren, bei denen die Umsetzungen mit Ethylenoxid bei niedrigen Temperaturen stattfinden, wegen der langen Verweilzeiten und der damit verbundenen niedrigen Raum-Zeit-Ausbeuten unrentabel.

WO 2001/53250 A1 (BP Chemicals Ltd.) betrifft ein kontinuierliches Verfahren zur Herstellung von TEOA, umfassend (i) einen Schritt der Synthetisierung des TEOAs durch das kontinuierliche Inkontaktbringen von Ammoniak mit Ethylenoxid unter Bedingungen, die die Bildung eines Reaktionsgemisches, umfassend Mono-, Di- und Triethanolamine, ermöglichen, (ii) einen Schritt der kontinuierlichen Trennung des Ammoniaks, das nicht umgesetzt wurde, aus dem Reaktionsgemisch, und (iii) einen Schritt der kontinuierlichen Trennung des TEOAs aus dem Gemisch, das aus Schritt (ii) resultiert, wobei das Verfahren dadurch gekennzeichnet ist, dass der letzte Schritt die Trennung des Monoethanolamins und etwas von dem Diethanolamin aus dem Gemisch, das aus Schritt (ii) resultiert, die Herstellung oder Isolierung eines speziellen Gemisches aus Alkanolaminen, umfassend TEOA und 0,5 bis 50 Gew.-% von zumindest einem sekundären Dialkanolamin, und die Trennung und Isolierung des TEOAs mit einem Reinheitsgrad von ≥ 99,2 Gew.-%, durch kontinuierliche Destillation des speziellen Gemisches aus Alkanolaminen umfasst.

WO 2005/035481 A2 (BASF AG) beschreibt die destillativen Abtrennung von Triethanolamin bei dem das Stoffgemisch in zwei Stufen destilliert wird. In der ersten Stufe werden die Leichtsiederfraktion und die Schwersiederfraktion entnommen und ausgeschleust und in der zweiten Stufe wird die Mittelsiederfraktion mit einem Gehalt an Triethanolamin von > 99,4 Gew.-% und Diethanolamin von < 0,2 Gew.-% destilliert. Die Destillation des Stoffgemischs erfolgt vorzugsweise in einer ersten Kolonne und einer mit dieser zusammengeschalteten zweiten Kolonne oder in einer Trennwandkolonne.

Der vorliegenden Erfindung lag die Aufgabe zugrunde, unter Überwindung von Nachteilen des Stands der Technik, ein verbessertes Verfahren zur Herstellung von Triethanolamin bereitzustellen, durch Abtrennung von Triethanolamin aus einem, insbesondere durch die Umsetzung von Ammoniak mit Ethylenoxid erhaltenen, Stoffgemisch. Für das TEOA sollte eine hohe Reinheit und Farbqualität, d.h. Verminderung der Verfärbung und/oder Verbesserung der Farbstabilität, insbesondere bei Lagerung, erreicht werden. Das Herstellverfahren sollte zudem besonders einfach und wirtschaftlich sein.

Demgemäß wurde ein Verfahren zur Herstellung von reinem Triethanolamin (TEOA) durch kontinuierliche destillative Auftrennung eines Ethanolamingemisches enthaltend TEOA und Diethanolamin (DEOA), wobei in einer Destillationskolonne (DEOA-Kolonne) DEOA abdestilliert wird und der anfallende Sumpfstrom enthaltend TEOA einer nachgeschaltenen Kolonne (TEOA-Kolonne) zugeführt wird, in der das reine TEOA als Seitenabzugsstrom entnommen wird, gefunden, welches dadurch gekennzeichnet ist, dass die Verweilzeit des Ethanolamingemisches im Sumpf der DEOA-Kolonne < 20 Minuten beträgt.

Erfindungsgemäß wurde erkannt, dass die Farbqualität, d.h. Verminderung der Verfärbung und/oder Verbesserung der Farbstabilität, insbesondere bei Lagerung, des Triethanolamines positiv beeinflusst werden kann, durch Verringerung der Verweilzeit des Ethanolamingemisches enthaltend Di- und Triethanolamin im Kolonnensumpf während der Abtrennung des Diethanolamines aus diesem Gemisch.

Die Erfindung verwendet bevorzugt ein Stoffgemisch, welches wie folgt erhalten wird. Zunächst wird, z. B. gemäß EP 673 920 A (BASF AG), durch die Umsetzung von wässrigem Ammoniak mit Ethylenoxid in flüssiger Phase unter erhöhtem Druck und erhöhter Temperatur in einem geeigneten Reaktor ein Ethanolamin-Gemisch, enthaltend die Hauptkomponenten Monoethanolamin (MEOA), Diethanolamion (DEOA) und Triethanolamin (TEOA), hergestellt.

Die Reaktionstemperaturen betragen hierbei insbesondere 110 bis 180 °C, bevorzugt 120 bis 150 °C, und die Drücke insbesondere 50 bis 150 bar (5 bis 15 MPa), bevorzugt 75 bis 120 bar (7,5 bis 12 MPa). Das molare Verhältnis von Ammoniak zu Ethylenoxid beträgt insbesondere 1 : 1 bis 100 : 1, bevorzugt 3 : 1 bis 50 : 1, besonders bevorzugt 4 : 1 bis 15 : 1, und der Ammoniak wird besonders als 60 bis 99,99 Gew.-%ige, bevorzugt 70 bis 95 Gew.-%ige, wässrige Lösung eingesetzt. Das eingesetzte Ethylenoxid kann als Gesamtmenge auf einmal oder in zwei bis zehn, bevorzugt zwei bis sechs, Teilmengen von jeweils 1 bis 99 Gew.-%, bevorzugt 5 bis 95 Gew.-%, z. B. 10 bis 70 Gew.-%, (jeweils bezogen auf die EO-Gesamtmenge) zugegeben werden.

Arbeitet man mit einem molaren Verhältnis von Ammoniak zu Ethylenoxid von mehr als 1 : 1, wird aus dem erhaltenen Produktgemisch anschließend in an sich bekannter Weise der überschüssige Ammoniak zusammen mit einem Teil des Wassers unter erhöhtem Druck (also > 1 bar (abs.)) und danach das restliche Wasser, vorzugsweise bei Normaldruck oder darunter, abdestilliert. Zurück bleibt ein im wesentlichen MEOA, DEOA und TEOA enthaltendes Rohprodukt mit einem Wassergehalt von bevorzugt kleiner 0,3 Gew.-%, besonders bevorzugt kleiner 0,1 Gew.-%.

Nach der sich anschließenden destillativen Abtrennung des Monoethanolamins (MEOA) in einer MEOA-Kolonne bei einem Absolutdruck von < 1 bar verbleibt ein Rohprodukt bestehend aus DEOA, TEOA und geringen Mengen an Nebenkomponenten, wie beispielsweise (2-(2-Hydroxyethoxy)ethyl)-di-(2-hydroxyethyl)-amin, (2-(2-Hydroxyethoxy)ethyl)-(2-hydroxyethyl)-amin und N,N'-Di-(2-hydroxyethyl)-piperazin. Ein typisches Rohgemisch enthält z. B. 50 bis 80 Gew.-% DEOA und 20 bis 50 Gew.-% TEOA.

Die Verweilzeit des Ethanolamingemisches im Sumpf der MEOA-Kolonne beträgt bevorzugt < 35 Minuten, z. B. im Bereich von 3 bis 30 Min., bevorzugt < 15 Minuten, z. B. im Bereich von 2 bis 10 Min., besonders bevorzugt < 7 Minuten, z. B. im Bereich von 1 bis 5 Min..

Für die Definition der Verweilzeit im Sumpf der MEOA-Kolonne gilt hier das Gleiche wie für die unten angegebene Definition der Verweilzeit im Sumpf der DEOA-Kolonne. Die MEOA-Kolonne wird so eingestellt, dass ein Rücklaufverhältnis im Bereich von insbesondere 0 bis 0,9, vorzugsweise von 0,1 bis 0,4, resultiert.

Die Zusammensetzung dieses Rohprodukts kann je nach dem ursprünglich eingesetzten molaren Verhältnis von Ammoniak zu Ethylenoxid schwanken.

In der Regel kann das erhaltene Ethanolamin-Gemisch, enthaltend überwiegend DEOA und TEOA, direkt einer fraktionierenden Destillation unterworfen werden, bei der nacheinander reines DEOA und TEOA erhalten werden.

Alternativ ist jedoch eine Vorgehensweise möglich, bei der dieses, überwiegend DEOA und TEOA enthaltende Rohprodukt, das einen Wassergehalt von bevorzugt kleiner 0,3 Gew.-%, insbesondere kleiner 0,1 Gew.-%, und einen Ammoniakgehalt von bevorzugt kleiner 0,1 % Gew.-%, insbesondere kleiner 0,01 Gew.-%, aufweist, mit 0,6 bis 1,2 Mol, bevorzugt 0,8 bis 1,1 Mol, Ethylenoxid (EO) pro Grammatom an Stickstoff gebundenen Wasserstoff im Rohprodukt bei Temperaturen von 110 bis 180 °C, bevorzugt 120 °C bis 180 °C, in flüssiger Phase umgesetzt wird. Diese Umsetzung erfolgt insbesondere wie in der GB 1 453 762 A beschrieben. Bevorzugt erfolgt die Umsetzung in Rohrreaktoren und mehrstufig, wobei beispielsweise in einer ersten Reaktionsstufe bei Temperaturen von vorzugsweise 125 bis 165 °C 50 bis 80 Gew.-% des eingesetzten Ethylenoxids, in einer zweiten Reaktionsstufe bei Temperaturen von vorzugsweise 150 bis 180 °C die restliche Menge des eingesetzten Ethylenoxids umgesetzt wird und in einer dritten Umsetzungsstufe die Reaktion bei Temperaturen von 120 bis 150 °C zu Ende geführt wird.

Das im erfindungsgemäßen Verfahren eingesetzte Ethanolamingemisch enthaltend DEOA und TEOA, bevorzugt enthaltend überwiegend DEOA und TEOA, weist besonders bevorzugt Gehalte an DEOA und TEOA wie folgt auf:
DEOA: 50 bis 80 Gew.-%, insbesondere 60 bis 75 Gew.-%.
TEOA: 20 bis 50 Gew.-%, insbesondere 25 bis 40 Gew.-%.

Das Ethanolamingemisch enthaltend DEOA und TEOA wird erfindungsgemäß kontinuierlich destillativ aufgetrennt. Hierfür eignen sich übliche Destillationsvorrichtungen. Solche Vorrichtungen sind einem Fachmann bekannt.

Zur Abtrennung einer überwiegenden Menge an DEOA aus dem Gemisch wird eine DEOA-Kolonne eingesetzt. Zur Abtrennung des TEOAs aus dem resltierenden Gemisch enthaltend DEOA-Reste wird eine nachgeschaltete TEOA-Kolonne eingesetzt. Bevorzugt können Destillationskolonnen mit wenigstens einer Quer- oder Längsunterteilung, ausgeführt in Form eines Bodens, einer Trennwand, geordneter Packungen oder Füllkörper, verwendet werden, wie z. B. beschrieben in WO 2005/035481 A2 (BASF AG).

Die sog. DEOA-Kolonne, in der zunächst das Diethanolamin vom Triethanolamin getrennt wird, wird mit einer Temperatur im Sumpf im Bereich von bevorzugt 170 °C bis 200 °C betrieben. Der gewählte Absolutdruck bewegt sich dabei bevorzugt bei Werten im Bereich von 10 mbar bis 20 mbar.

Druckangaben für Destillationskolonnen beziehen sich in diesem Dokument stets auf den Absolutdruck im Kolonnenkopf.

Die Kolonne wird so eingestellt, dass ein Rücklaufverhältnis im Bereich von insbesondere 0 bis 0,6, vorzugsweise von 0,25 bis 0,4, resultiert.

Definition ,Rücklaufverhältnis': Massenverhältnis von in die Kolonne zurückgeführtem und als Destillat abgezogenem Kondensat am Kopf einer Destillationskolonne.

Das Ethanolamingemisch enthaltend DEOA und TEOA wird vorzugsweise seitlich, besonders seitlich im mittleren Kolonnenbereich, in die DEOA-Kolonne eingespeist.

Erfindungsgemäß beträgt die Verweilzeit des Ethanolamingemisches im Sumpf der DEOA-Kolonne < 20 Minuten, z. B. im Bereich von 1 bis 18 Min., bevorzugt < 15 Minuten, z. B. im Bereich von 2 bis 13 Min., besonders bevorzugt < 12 Minuten, z. B. im Bereich von 3 bis 11 Min.

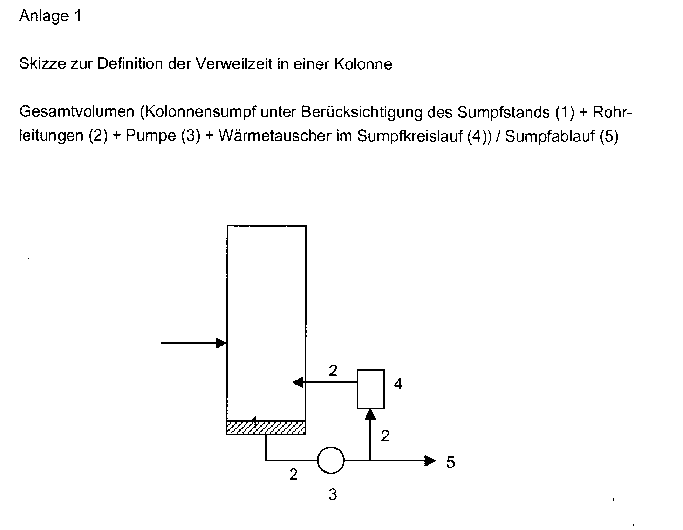

Als Verweilzeit im Sumpf der DEOA-Kolonne wird die Zeit verstanden, die sich ergibt, wenn man die tatsächlichen Flüssigkeitsvolumina von Kolonnensumpf, Rohrleitungen und Pumpe im Sumpfkreislauf und Hold-up im Verdampfer/Wärmetauscher addiert und durch den Volumenstrom, der diesem Sumpfkreislauf entnommen wird, dividiert. Die Zeichnung der Anlage 1 dient diesbezüglich der weiteren Erläuterung.

Das Diethanolamin wird aus der DEOA-Kolonne vorzugsweise als Seitenabzugsstrom isoliert und hat bevorzugt eine Reinheit von ≥ 99,3 Gew.-%.

Der Sumpf der DEOA-Kolonne, der zur nachgeschalteten TEOA-Kolonne geführt wird, enthält besonders im Bereich von 15 bis 33 Gew.-% DEOA, 65 bis 83 Gew.-% TEOA und 0,1 bis 2 Gew.-% sonstige Nebenkomponenten.

Die TEOA-Kolonne, in der das Triethanolamin isoliert wird, wird mit einer Temperatur im Sumpf von bevorzugt im Bereich von 170 °C bis 200 °C betrieben. Der gewählte Absolutdruck bewegt sich dabei bevorzugt bei Werten von 1 mbar bis 10 mbar.

Die Kolonne wird so eingestellt, dass ein Rücklaufverhältnis im Bereich von insbesondere 0 bis 0,6, vorzugsweise von 0,1 bis 0,4, resultiert.

Das aufzutrennende Ethanolamingemisch enthaltend DEOA und TEOA wird vorzugsweise seitlich, besonders seitlich im mittleren Kolonnenbereich, in die TEOA-Kolonne eingespeist.

Eine Leichtsiederfraktion enthaltend ein Gemisch vom Diethanolamin (60 bis 100 Gew.-%, besonders 70 bis 95 Gew.-%) und Triethanolamin (0 bis 40 Gew.-%, besonders 5 bis 30 Gew.-%) wird am Kolonnenkopf abgetrennt und ggf. zurück in die DEOA-Kolonne geleitet.

Die Schwersiederfraktion besteht aus Triethanolamin (50 bis 100 Gew.-%, besonders 80 bis 98 Gew.-%, TEOA) und Nebenkomponenten und wird am Kolonnensumpf entnommen und ausgeschleust.

Die verbleibende Mittelsiederfraktion mit einem Gehalt an Triethanolamin von ≥ 99,3 Gew.-%, besonders ≥ 99,4 Gew.-%, wird, bevorzugt im mittleren Teil der TEOA-Kolonne, als Seitenabzugsstrom isoliert.

Aufgrund der Temperaturempfindlichkeit des Ethanolamin-Stoffgemischs ist es vorteilhaft, die TEOA-Kolonne mit einem Verdampfer zu betreiben, der eine geringe Wandtemperatur sowie einen kleinen Flüssigkeitsinhalt aufweist. Insgesamt hat es sich als besonders günstig erwiesen, einen Fallfilmverdampfer zu verwenden. Der Kolonnensumpf und der Verdampfersumpf werden dabei so gestaltet, dass die Verweilzeit der Schwersiederfraktion im Sumpf de TEOA-Kolonne im Bereich von 10 bis 180 Min., bevorzugt 30 bis 150 Min., beträgt. Bei diesen Verweilzeiten wird ein Optimum aus der Abtrennung der Mittelsiederfraktion und der Vermeidung der Bildung unerwünschter Nebenprodukte erzielt.

Für die Definition der Verweilzeit im Sumpf der TEOA-Kolonne gilt hier das Gleiche wie für die oben angegebene Definition der Verweilzeit im Sumpf der DEOA-Kolonne.

In einer vorteilhaften Ausführungsform wird das in der TEOA-Kolonne im Seitenabzug erhaltene TEOA zur weiteren Reinigung und/oder Verbesserung der Farbqualität in einer weiteren nachgeschalteten Kolonne destilliert und dort über Kopf abgetrennt, insbesondere wie in WO 2005/035481 A2 (BASF AG) beschrieben. Die im Sumpf der nachgeschalteten Kolonne anfallenden Hochsieder werden bevorzugt in den mittleren Bereich der TEOA-Kolonne zurückgeführt. Das Rücklaufverhältnis in der nachgeschalteten Kolonne liegt bevorzugt im Bereich von 0,2 und 0,7. Vorzugsweise werden die TEOA-Kolonne und die nachgeschaltete Kolonne mit gleichen oder annähernd gleichen Temperaturprofilen betrieben.

In einer weiteren vorteilhaften Ausführungsform stellt die TEOA-Kolonne eine Trennwandkolonne dar, insbesondere wie in WO 2005/035481 A2 (BASF AG) beschrieben. Eine Trennwandkolonne ist im Prinzip eine apparative Vereinfachung von zwei thermisch gekoppelten Destillationskolonnen. Sie enthält in der Regel eine senkrecht angeordnete und sich oberhalb und unterhalb der Zulaufstelle erstreckende Trennwand, welche die Kolonne in einen Zulaufteil und einen Entnahmeteil unterteilt. Die Trennwandkolonne kann als Füllkörper oder geordnete Packungen enthaltende Packungskolonne oder als Bodenkolonne ausgestaltet sein. Das Stoffgemisch wird im mittleren Bereich der Trennwand in die Kolonne eingeleitet. Die erste Destillationsstufe wird im Zulaufteil der Kolonne durchgeführt und die nach Entnahme der Leichtsiederfraktion am Kolonnenkopf und Schwersiederfraktion am Kolonnensumpf verbleibende Mittelsiederfraktion wird im Entnahmeteil der Kolonne destilliert, wobei der Austrag von reinem Triethanolamin (TEOA) über einen Seitenabzug im mittleren Bereich der Trennwand (gegenüber der Einleitungsstelle) erfolgt und die in der zweiten Destillationsstufe entstehenden Hochsieder ebenfalls im Kolonnensumpf ausgeschleust werden.

In weiteren Ausführungsformen kann man mehrere DEOA-Kolonnen und TEOA-Kolonnen (jeweils z. B. 2 oder 3) betreiben und beliebig verschalten. Der Austrag aus der DEOA-Kolonne kann entweder direkt in die TEOA-Kolonne gefahren werden oder auch zunächst zwischengelagert werden.

Das erfindungsgemäße Verfahren liefert TEOA besonders in einer Reinheit von ≥ 99,3 Gew.-%, weiter besonders ≥ 99,4 Gew.-%, insbesondere ≥ 99,5 bis 99,8 Gew.-%.

Die APHA-Farbzahl von erfindungsgemäß erhaltenem TEOA beträgt besonders ≤ 100, weiter besonders ≤ 90, und liegt ganz besonders im Bereich von 30 bis 80.

TEOA in besonders guter Farbqualität, besonders mit einer APHA-Farbzahl ≤ 20 und hoher Farbstabilität, wird erhalten, wenn man, wie insbesondere in EP 4015 A und EP 1 132 371 A beschrieben, eine wirksame Menge an phosphoriger oder unterphosphoriger Säure oder einem Derivat einer dieser Säuren (z. B. einem Salz, z. B. Natriumsalz) vor oder während der Ethanolamin-Synthese aus Ethylenoxid und Ammoniak und/oder im Laufe der destillativen Auftrennung des erhaltenen EthanolaminGemisches zugibt. Vorzugsweise erfolgt die Zugabe erst vor der TEOA-Reindestillation in der TEOA-Kolonne. Erfolgt die Zugabe des Additivs nach der Umsetzung von Ethylenoxid und Ammoniak in der Destillation der daraus erhaltenen Ethanolamine, beträgt die zudosierte Menge bevorzugt im Bereich von 0,005 bis 2 Gew.-% bezogen auf die Summe der Ethanolamine (MEOA, DEOA, TEOA), im Zulauf zur betreffenden Kolonne.

Die APHA-Farbzahl von derart erfindungsgemäß erhaltenem TEOA beträgt besonders ≤ 20, weiter besonders ≤ 15, und liegt ganz besonders im Bereich von 1 bis 10.

### Beispiele

Die Ermittlung der APHA-Farbzahl erfolgt gemäß DIN-ISO 6271.

### Beispiel 1 a

Ein Ethanolamingemisch, enthaltend die Hauptkomponenten Monoethanolamin (MEOA), Diethanolamion (DEOA) und Triethanolamin (TEOA), wurde durch die Umsetzung von wässrigem Ammoniak mit Ethylenoxid in flüssiger Phase unter erhöhtem Druck und erhöhter Temperatur in einem geeigneten Reaktor hergestellt. Das erhaltene Ethanolamin-Gemisch wurde nach Abtrennung von NH₃, Wasser und MEOA in eine DEOA-Kolonne geführt, um DEOA zu isolieren. Der Zulauf zur DEOA-Kolonne enthielt 1,6 Gew.-% MEOA, 70,2 Gew.-% DEOA und 28,2 Gew.-% TEOA. Die DEOA-Kolonne wurde bei einem Absolutdruck von 14 mbar und mit einer Kopftemperatur von 135 °C und mit einer Sumpftemperatur von 189 °C betrieben; das Rücklaufverhältnis betrug 0,33.

Die Verweilzeit des Gemisches im Sumpf der DEOA-Kolonne wurde auf 18 Minuten eingestellt.

Als Kopfprodukt wurde DEOA mit einer Reinheit von 99,5 Gew.-% erhalten.

Das Sumpfprodukt (23 Gew.-% DEOA, 76 Gew.-% TEOA, 1 Gew.-% Nebenkomponenten) wurde danach in die TEOA-Kolonne geführt, die bei einem Absolutdruck von 1,3 mbar und mit einer Kopftemperatur von 71 °C und mit einer Sumpftemperatur von 185 °C betrieben wurde. Das Rücklaufverhältnis betrug 0,22.

Im Zulauf der TEOA-Kolonne wurde phosphorige Säure (0,12 Gew.-% bezogen auf den Zulauf der Kolonne) zugemischt. Das Kopfprodukt der TEOA-Kolonne wurde in die DEOA-Kolonne zurückgeführt. Der Seitenablauf wurde in eine weitere nachgeschaltete Kolonne geführt. Am Kopf der nachgeschalteten Kolonne wurde TEOA mit einer Reinheit von 99,52 Gew.-% und einer Farbzahl von 20 APHA isoliert.

### Beispiel 1 b

Unter den gleichen Bedingungen wie oben beschrieben, aber mit einer reduzierten Verweilzeit des Gemisches im Sumpf der DEOA-Kolonne von 7 Minuten, wurde TEOA mit einer Reinheit von 99,62 Gew.-% und ein Farbzahl von 6 APHA erhalten.

Die Verweilzeit wurden jeweils wie oben beschrieben errechnet.

Die Beispiele zeigen, dass durch Reduzierung der Verweilzeit des Sumpfgemisches in der DEOA-Kolonne und damit eine Reduzierung der thermischen Belastung des TEOAs während der Abtrennung der verschiedenen Ethanolamine, ein positiver Einfluss auf die Farbqualität, insb. Farbzahl, des Triethanolamins erreicht wird. Wenn die Verweilzeit im Sumpf der DEOA-Kolonne insbesondere auf < 20 Minuten, besonders bevorzugt < 12 Minuten reduziert wird, wird ein deutlich weniger gefärbtes Triethanolamin erhalten.

### Beispiel 2

Folgendes Beispiel illustriert, dass eine lange thermische Belastung eines Ethanolamingemisches aus dem Kolonnensumpf der DEOA-Kolonne, wie sie durch eine erhöhte Verweilzeit im Kolonnensumpf auftritt, zum Verlust von Wertprodukt und einem deutlichen Anstieg der Farbzahl führt. Dadurch wird die Aufreinigung von TEOA in der nachfolgenden Kolonne deutlich erschwert.

Das Sumpfprodukt einer DEOA-Kolonne wurde analysiert: 17,0 Gew.-% DEOA, 81,4 Gew.-% TEOA, 1,6 Gew.-% Nebenkomponenten, Farbzahl (APHA) 17. Dieses Gemisch wurde unter Stickstoff für 1 h bei 190 °C erhitzt. Der Anteil an Nebenkomponenten stieg auf 4,0 Gew.-% an und die Farbzahl (APHA) erhöhte sich auf 120.

### Beispiel 3

Eine TEOA-Kolonne mit Seitenabzugskolonne nach WO 2005/035481 A wurde mit einem Gemisch aus DEOA, TEOA und Nebenkomponenten gemäß untenstehender Auflistung (Zulauf 1 und 2) beschickt. Diese Zuläufe waren Sumpfprodukte einer DEOA-Destillation, die bei unterschiedlichen Verweilzeiten im Sumpf der DEOA-Kolonne erhalten wurden. Die Verweilzeit wurde jeweils wie oben beschrieben errechnet.

Zulauf 1: 24 Gew.-% DEOA, 72 Gew.-% TEOA, 4 Gew.-% Nebenkomponenten, erhalten aus einer DEOA-Destillation bei ca. 180 °C mit > 30 Min. und < 70 Min. Verweilzeit im Sumpf der Kolonne.

Zulauf 2: 17 Gew.-% DEOA, 79 Gew.-% TEOA, 4 Gew.-% Nebenkomponenten, erhalten aus einer DEOA-Destillation bei 175 bis 180 °C Sumpftemperatur mit 5 bis 10 Min. Verweilzeit im Sumpf der Kolonne.

Die TEOA-Kolonne wurde bei 4 mbar (abs.) Kopfdruck, 195 °C Sumpftemperatur, 160 °C Kopftemperatur und einem Rücklaufverhältnis am Kopf der Kolonne (Rücklaufverhältnis nur hier wie folgt definiert: Rücklaufmenge (Liter) / gesamter Kopfablaufmenge (Liter)) von 0,1 unter Zudosierung von 0,05 Gew.-% phosphoriger Säure (bezogen auf den Kolonnenzulauf) betrieben. TEOA wurde im Kopf einer nachgeschalteten Kolonne (Seitenabzugskolonne) isoliert.

Mit Zulauf 1 wurde TEOA mit einer Reinheit von 98,7 Gew.-% und einer Farbzahl (APHA) von 17 erhalten.

Mit Zulauf 2 wurde TEOA mit einer Reinheit von 99,4 Gew.-% und einer Farbzahl (APHA) von 3 erhalten.

Das Beispiel zeigt: Eine lange Verweilzeit im Sumpf der DEOA-Kolonne beeinflusst die nachfolgende TEOA-Destillation negativ und führt zu einer TEOA-Qualität mit geringerer Reinheit und schlechterer Farbzahl.

## Patentansprüche

1. Verfahren zur Herstellung von reinem Triethanolamin (TEOA) durch kontinuierliche destillative Auftrennung eines Ethanolamingemisches enthaltend TEOA und Diethanolamin (DEOA), wobei in einer Destillationskolonne (DEOA-Kolonne) DEOA abdestilliert wird und der anfallende Sumpfstrom enthaltend TEOA einer nachgeschaltenen Kolonne (TEOA-Kolonne) zugeführt wird, in der das reine TEOA als Seitenabzugsstrom entnommen wird, **dadurch gekennzeichnet, dass** die Verweilzeit des Ethanolamingemisches im Sumpf der DEOA-Kolonne < 20 Minuten beträgt.

2. Verfahren nach Anspruch 1, **dadurch gekennzeichnet, dass** die Verweilzeit des Ethanolamingemisches im Sumpf der DEOA-Kolonne < 15 Minuten beträgt.

3. Verfahren nach Anspruch 1, **dadurch gekennzeichnet, dass** die Verweilzeit des Ethanolamingemisches im Sumpf der DEOA-Kolonne im Bereich von 3 bis < 12 Minuten beträgt.

4. Verfahren nach einem der vorhergehenden Ansprüche, **dadurch gekennzeichnet, dass** das DEOA in der DEOA-Kolonne als Seitenabzugsstrom entnommen wird.

5. Verfahren nach dem vorhergehenden Anspruch, **dadurch gekennzeichnet, dass** das DEOA in einer Reinheit von ≥ 99,3 Gew.-% entnommen wird.

6. Verfahren nach einem der vorhergehenden Ansprüche, **dadurch gekennzeichnet, dass** das TEOA der TEOA-Kolonne in einer Reinheit von ≥ 99,3 Gew.-% entnommen wird.

7. Verfahren nach einem der vorhergehenden Ansprüche, **dadurch gekennzeichnet, dass** am Kopf der TEOA-Kolonne ein Produktstrom enthaltend ≥ 60 Gew.% DEOA und ≤ 40 Gew.-% TEOA entnommen wird.

8. Verfahren nach dem vorhergehenden Anspruch, **dadurch gekennzeichnet, dass** der am Kopf der TEOA-Kolonne entnommene Produktstrom in die DEOA-Kolonne zurückgeführt wird.

9. Verfahren nach einem der vorhergehenden Ansprüche, **dadurch gekennzeichnet, dass** das in der TEOA-Kolonne im Seitenabzug erhaltene TEOA in einer weiteren nachgeschalteten Kolonne destilliert und dort über Kopf abgetrennt wird.

10. Verfahren nach einem der vorhergehenden Ansprüche, **dadurch gekennzeichnet, dass** die TEOA-Kolonne eine Trennwandkolonne darstellt.

11. Verfahren nach einem der vorhergehenden Ansprüche, **dadurch gekennzeichnet, dass** das aufzutrennende Ethanolamingemisch enthaltend TEOA und DEOA zuvor durch Umsetzung von Ammoniak mit Ethylenoxid in flüssiger Phase und anschließender Abtrennung von NH₃, Wasser und Monoethanolamin (MEOA) erhalten wurde.

12. Verfahren nach einem der vorhergehenden Ansprüche, **dadurch gekennzeichnet, dass** man dem aufzutrennenden Ethanolamingemisch eine wirksame Menge an phosphoriger oder unterphosphoriger Säure oder einem Derivat einer dieser Säuren zugibt.

13. Verfahren nach dem vorhergehenden Anspruch zur Herstellung von TEOA mit einer APHA-Farbzahl von ≤ 20.

## Claims

1. A process for preparing pure triethanolamine (TEOA) by continuously distillatively separating an ethanolamine mixture comprising TEOA and diethanolamine (DEOA), by distilling off DEOA in a distillation column (DEOA column) and supplying the resulting bottom stream comprising TEOA to a downstream column (TEOA column) in which the pure TEOA is withdrawn as a side draw stream, wherein the residence time of the ethanolamine mixture in the bottom of the DEOA column is < 20 minutes.

2. The process according to claim 1, wherein the residence time of the ethanolamine mixture in the bottom of the DEOA column is < 15 minutes.

3. The process according to claim 1, wherein the residence time of the ethanolamine mixture in the bottom of the DEOA column is in the range from 3 to < 12 minutes.

4. The process according to any one of the preceding claims, wherein the DEOA is withdrawn in the DEOA column as a side draw stream.

5. The process according to the preceding claim, wherein the DEOA is withdrawn in a purity of ≥ 99.3% by weight.

6. The process according to any one of the preceding claims, wherein the TEOA of the TEOA column is withdrawn in a purity of ≥ 99.3% by weight.

7. The process according to any one of the preceding claims, wherein a product stream comprising ≥ 60% by weight of DEOA and ≤ 40% by weight of TEOA is withdrawn at the top of the TEOA column.

8. The process according to the preceding claim, wherein the product stream withdrawn at the top of the TEOA column is recycled into the DEOA column.

9. The process according to any one of the preceding claims, wherein the TEOA obtained in the side draw in the TEOA column is distilled in a further downstream column and removed there via the top.

10. The process according to any one of the preceding claims, wherein the TEOA column is a dividing wall column.

11. The process according to any one of the preceding claims, wherein the ethanolamine mixture comprising TEOA and DEOA for separation has been obtained beforehand by reacting ammonia with ethylene oxide in the liquid phase and subsequently removing NH₃, water and monoethanolamine (MEOA).

12. The process according to any one of the preceding claims, wherein an effective amount of phosphorous acid or hypophosphorous acid or a derivative of one of these acids is added to the ethanolamine mixture for separation.

13. The process according to the preceding claim for preparing TEOA with an APHA color number of ≤ 20.

## Revendications

1. Procédé pour la préparation de triéthanolamine (TEOA) pure par séparation continue par distillation d'un mélange d'éthanolamines contenant de la TEOA et de la diéthanolamine (DEOA), la DEOA étant éliminée par distillation dans une colonne de distillation (colonne DEOA) et le flux de fond produit, contenant de la TEOA, étant introduit dans une colonne disposée en aval (colonne TEOA) de laquelle la TEOA pure est prélevée sous forme de flux de soutirage latéral, **caractérisé en ce que** le temps de séjour du mélange d'éthanolamines dans le fond de la colonne DEOA est < 20 minutes.

2. Procédé selon la revendication 1, **caractérisé en ce que** le temps de séjour du mélange d'éthanolamines dans le fond de la colonne DEOA est < 15 minutes.

3. Procédé selon la revendication 1, **caractérisé en ce que** le temps de séjour du mélange d'éthanolamines dans le fond de la colonne DEOA de situe dans la plage de 3 à < 12 minutes.

4. Procédé selon l'une quelconque des revendications précédentes, **caractérisé en ce que** la DEOA dans la colonne de DEOA est prélevée sous forme de flux de soutirage latéral.

5. Procédé selon la revendication précédente, **caractérisé en ce que** la DEOA est prélevée avec une pureté ≥ 99,3% en poids.

6. Procédé selon l'une quelconque des revendications précédentes, **caractérisé en ce que** la TEOA est prélevée de la colonne de TEOA avec une pureté ≥ 99,3% en poids.

7. Procédé selon l'une quelconque des revendications précédentes, **caractérisé en ce qu'**on prélève, en tête de la colonne TEOA, un flux de produits contenant ≥ 60% en poids de DEOA et ≤ 40% en poids de TEOA.

8. Procédé selon la revendication précédente, **caractérisé en ce que** le flux de produits prélevé en tête de la colonne TEOA est recyclé dans la colonne DEOA.

9. Procédé selon l'une quelconque des revendications précédentes, **caractérisé en ce que** la TEOA obtenue dans la colonne TEOA dans le soutirage latéral est distillée dans une autre colonne disposée en aval et y est séparée via la tête.

10. Procédé selon l'une quelconque des revendications précédentes, **caractérisé en ce que** la colonne TEOA est une colonne à paroi de séparation.

11. Procédé selon l'une quelconque des revendications précédentes, **caractérisé en ce que** le mélange d'éthanolamines à séparer contenant de la TEOA et de la DEOA a été obtenu au préalable par transformation d'ammoniaque avec de l'oxyde d'éthylène en phase liquide et séparation consécutive de NH₃, d'eau et de monoéthanolamine (MEOA).

12. Procédé selon l'une quelconque des revendications précédentes, **caractérisé en ce qu'**on ajoute au mélange d'éthanolamines à séparer une quantité active d'acide phosphoreux ou hypophosphoreux ou d'un dérivé d'un de ces acides.

13. Procédé selon la revendication précédente, pour la préparation de TEOA présentant un indice de couleur APHA ≤ 20.
